# FASCICULE DE BREVET EUROPEEN

(11) **EP 0 744 028 B1**
(45) Date de publication et mention de la délivrance du brevet: **03.09.2003**
(21) Numéro de dépôt: 95909007.7
(22) Date de dépôt: 10.02.1995
(51) Int. Cl.: G01N 33/543, G01N 33/545, G01N 33/547, C12Q 1/68

(54) **SURFACES HAUTEMENT SPECIFIQUES POUR REACTIONS BIOLOGIQUES, PROCEDE POUR LEUR PREPARATION ET PROCEDE POUR LEUR UTILISATION**
FESTPHASEN, SPEZIFISCH FÜR BIOLOGISCHE REAKTIONEN, IHRE HERSTELLUNG UND ANWENDUNG
HIGHLY SPECIFIC SURFACES FOR BIOLOGICAL REACTIONS, METHOD OF PREPARATION AND UTILIZATION

(30) Priorité: 11.02.1994 FR 9401574; 17.06.1994 FR 9407444
(43) Date de publication de la demande: 27.11.1996
(73) Titulaire: INSTITUT PASTEUR, 75015 Paris (FR); CENTRE NATIONAL DE LA RECHERCHE SCIENTIFIQUE, 75016 Paris Cédex (FR)
(72) Inventeur: BENSIMON, David, F-75013 Paris (FR); BENSIMON, Aaron, F-92160 Antony (FR); HESLOT, François, F-78220 Viroflay (FR)
(74) Mandataire: Ahner, Francis
(86) Numéro de dépôt international: FR9500164
(87) Numéro de publication internationale: WO95022056

(56) Documents cités:
- EP-A- 0 125 995
- EP-A- 0 127 438
- EP-A- 0 350 407
- EP-A- 0 388 940
- EP-A- 0 391 674
- EP-A- 0 435 785
- EP-A- 0 578 148
- DE-A- 3 717 210
- US-A- 5 102 798
- NUCLEIC ACIDS RESEARCH., vol. 16,no. 22, 1988 ARLINGTON, VIRGINIA US, pages 10861-10880, V. LUND ET AL. cité dans la demande

## Description

La présente invention concerne notamment des surfaces très hautement spécifiques utilisables en biologie ainsi que leurs applications et des procédés pour leur préparation.

On connaît depuis longtemps la très grande spécificité et la très grande sélectivité de certaines réactions biologiques, notamment les réactions antigènes/anticorps, les réactions d'hybridation d'ADN ou d'ARN, les réactions interprotéines ou de type avidine/streptavidine/biotine, de même que les réactions des ligands et de leurs récepteurs.

On sait maintenant tirer partie de ces spécificités, notamment pour mettre en évidence la présence ou l'absence de l'un des éléments du couple réactionnel dans un échantillon ou bien éventuellement pour séparer l'un des éléments du couple d'un milieu plus complexe.

Toutefois, lorsque l'on souhaite détecter la présence d'une molécule à très faible concentration dans un milieu très complexe, les procédés actuellement connus donnent parfois des résultats très aléatoires, compte tenu notamment du problème des bruits de fond intervenant lors des étapes de séparation et/ou de détection.

C'est pourquoi, ce qui sera appelé ci-après "pêche moléculaire", c'est-à-dire la possibilité de pouvoir mettre en évidence chacune des molécules qui sont recherchées lorsqu'elles sont à des concentrations très faibles, n'a jusqu'ici pas été possible.

A titre d'exemple, l'analyse d'un échantillon d'ADN passe par l'utilisation d'une sonde dite d' "hybridation" correspondant à la séquence complémentaire de la séquence recherchée. Dans ces conditions, le problème posé est d'isoler l'hybride du milieu et de détecter avec un bon rapport signal/bruit (S/N) le nombre éventuellement réduit de réactions positives.

C'est pourquoi on utilise maintenant, dans la plupart des cas, une étape intermédiaire destinée à amplifier la séquence que l'on cherche à détecter, par exemple en utilisant la méthode PCR ou des méthodes d'amplification conduisant aux mêmes résultats, dans ces conditions on augmente la concentration de la séquence à déterminer dans l'échantillon et cette détection est évidemment beaucoup plus commode.

Cependant, l'étape d'amplification est sensible aux contaminants et conduit à des erreurs qui lui sont propres.

Il serait donc préférable, dans la mesure du possible, de pouvoir détecter la présence de la séquence d'acide nucléique sans phase d'amplification.

On a proposé d'utiliser, de façon à mettre en évidence la réaction d'hybridation spécifique, une étape intermédiaire d'ancrage du produit d'hybridation sur une surface solide présentant certaines spécificités. Par exemple, il est possible d'utiliser certaines surfaces prétraitées permettant de fixer certaines protéines ou de l'ADN, qu'il ait été ou non modifié.

De telles surfaces sont commercialement disponibles (Covalink, Costar, Estapor, Bangs, Dynal par exemple) sous forme de billes ou de puits présentant à leur surface des groupements COOH, NH₂ ou OH par exemple.

Il a été proposé également pour obtenir de tels groupements d'utiliser une étape intermédiaire présentant un groupement vinyl qui est ensuite oxydé pour présenter des groupes COOH ou OH (USA 4.539.061 et EP 435 785).

EP 0 388 940 décrit des particules magnétiques à base de polyacrylamide ou de polystyrene recouvertes par une couchge monomoléculaire comprenant une partie hydrophile (radicaux COOH, NH₂, OH...) et une partie hydrophobe (oléfine telle que vinylène ou vinylidène). Ces particules permettent l'absorption d'anticorps et sont utilisées dans des tests d'immuno-agglutination.

On peut alors fonctionnaliser l'ADN avec un groupement réactif, amine par exemple, et procéder à une réaction avec ces surfaces. Ces méthodes nécessitent cependant une fonctionnalisation particulière de l'ADN à fixer.

On a également décrit une technique permettant l'ancrage sans traitement préalable de l'ADN. Ce procédé consiste à faire réagir le phosphate libre de l'extrémité 5' de la molécule avec une amine secondaire (surface NH Covalink).

On peut aussi fixer l'ADN à un groupement ou une protéine P₀ pour la faire réagir avec une suface recouverte d'un groupement ou une protéine P₁, susceptible de réagir spécifiquement avec P₀. Le couple P₀/P₁ peut être un couple de type biotine/streptavidine ou digoxigénine/anticorps dirigé contre la digoxigénine, (anti-DIG) par exemple.

De telles surfaces sont, cependant, dans la plupart des cas insuffisamment spécifiques (V. Lund et al., Nucl. Acids Res., 16, 1861 (1988)). Ainsi, la présence d'interactions parasites, même faibles, de type adsorption non-spécifique conduit à des adsorptions efficaces pour des molécules longues, capables de contracter avec le solide un grand nombre de points d'interaction faible. Ces surfaces conduisent à des applications potentielles manquant de sensibilité et/ou avec un grand taux de bruit de fond dans le cas d'un petit nombre de molécules à pêcher. De plus, certaines de ces surfaces présentent un fort taux de fluorescence parasite potentiellement gênante lors de la phase de détection.

Pour ce qui concerne la détection proprement dite, en particulier pour la mise en évidence de l'ADN, le brevet Français 78 10975 décrit un procédé couplant la sonde à une enzyme qui permet la révélation à l'aide d'un substrat chromogène. Il est, en outre, possible de quantifier la réaction par une mesure de colorimétrie.

Une telle technique n'est cependant pas adaptée directement à la détection de traces, c'est pourquoi, là aussi, elle doit être précédée dans la plupart des cas d'une étape d'amplification de la quantité d'acide nucléique recherchée, par exemple par la méthode PCR.

Ce procédé de détection dit "par sonde froide" a été développé pour éviter l'utilisation de marqueurs radioactifs qui donnent des résultats qui en sensibilité sont voisins mais qui évidemment présentent des problèmes de manipulation, compte tenu de la présence de produits radioactifs et des problèmes de temps de révélation longs si on cherche une grande sensibilité.

Pour certaines applications particulières, notamment des méthodes dérivées de l'imagerie ex-vivo, on a proposé une méthode directe d'observation de la réaction en couplant le produit de l'hybridation à des microbilles, notamment de PMMA, convenablement traitées chimiquement à leur surface. La méthode repose sur l'identification directe sous microscope à balayage électronique de la présence de ces microbilles d'un diamètre typique de 60 nm et de plus repose sur les techniques d'ancrage sur solides connues mais insuffisamment spécifiques, ainsi qu'il a été décrit plus haut.

Les techniques ci-dessus ne sont évidemment pas limitées à la détection d'acides nucléiques. Dans le même esprit, on a proposé par exemple la détection d'anticorps. Il s'agit des tests de type ELISA que nous ne redécrirons pas ici et qui, pour résumer, permettent de coupler la présence d'un anticorps à un ancrage associé d'une molécule d'antigène sur un solide. A nouveau, les problèmes de spécificité et de réactions parasites se posent. La phase de détection peut se baser ensuite sur un couplage à une réaction chromogène ayant ses propres problèmes de sensibilité.

En résumé, les méthodes de la technique antérieure ou leurs combinaisons présentent un certain nombre d'inconvénients, notamment :
- soit d'être potentiellement dangereuses par mise en oeuvre de produits radioactifs,
- soit de nécessiter des temps de révélation trop longs,
- soit d'être gênées par des problèmes spécifiques au niveau de la phase d'amplification,
- soit de passer par des surfaces solides insuffisamment spécifiques,
- soit d'être trop peu sensibles,
- soit, enfin, de nécessiter en plus de la phase d'accrochage sur un solide l'utilisation peu commode, évidemment, d'un microscope électronique.

Enfin, dans la plupart des cas, les procédés connus ne permettent pas de reconnaître sur une molécule donnée la position spécifique du motif recherché. Or ce type de reconnaissance est important lorsque l'on cherche à faire une cartographie, en particulier dans le cadre de la cartographie du génome, on cherche à connaître dans un premier temps la position spatiale approximative par rapport à une extrémité de la molécule d'un gène donné sur un ADN ou un ARN.

La présente invention qui se propose de remédier aux inconvénients des procédés antérieurs repose sur l'utilisation de surfaces très hautement spécifiques qui lors de leur mise en oeuvre conduisent à des bruits de fond excessivement limités, en particulier du fait qu'elles éliminent les fixations parasites.

Plus particulièrement, la présente invention concerne une surface hautement spécifique pour réactions biologiques, caractérisée en ce qu'elle comporte un support présentant en surface au moins une couche essentiellement compacte d'un composé organique présentant, à l'extérieur de la couche, un groupement exposé comportant une double liaison ethylénique, notamment un groupement vinyl, ayant une affinité pour un type de molécule à activité biologique dans certaines conditions de réactions, notamment de pH ou de teneurs ioniques, les autres éléments de la couche étant essentiellement inaccessibles pour lesdites molécules dans lesdites conditions de réaction.

Par "affinité", il faut entendre ici, aussi bien une réactivité chimique qu'une adsorption d'un type quelconque, ceci dans les conditions de fixation desdites molécules biologiques.

Par "support", on entend désigner aussi bien un support solide qu'un support constitué par un élément non solide tel qu'une particule liquide ou gazeuse présentant, notamment, une couche compacte telle que décrite ci-dessus.

La surface est "essentiellement compacte", c'est-à-dire qu'elle limite l'accès de la molécule à activité biologique aux couches inférieures et/ou au support, étant entendu que des défauts de couverture de la surface sont tolérables.

Ces surfaces hautement spécifiques pour réactions biologiques, comportent un support présentant en surface des groupements à double liaison, notamment vinyl (-CH=CH₂, ci-après surfaces C = C) accessibles à la solution. Elles sont capables d'ancrer directement des molécules d'intérêt biologique (ADN, ARN, PNA, Protéines, lipides, saccharides) dans certaines conditions de pH ou de teneur ionique du milieu. En particulier, ces surfaces ne nécessitent pas de modification chimique particulière ni de la surface, ni des molécules biologiques à ancrer. Il n'existe pas de documents mentionnant une telle utilisation de surface à groupements vinyl.

Par "ancrage", il faut entendre ici une fixation par lien covalent résultant d'une réactivité chimique, ou lien non covalent résultant d'interactions physico-chimiques telles qu'une adsorption de type quelconque, ceci dans les conditions de pH ou de teneur ionique du milieu de fixation desdites molécules biologiques.

Les surfaces selon la présente invention peuvent être obtenues par la mise en oeuvre de divers procédés. On peut citer à titre d'exemple :
(A) une couche de polymère carboné, éventuellement branché, d'au moins 1 nm d'épaisseur, présentant :
   . des groupements comportant une double liaison éthylénique,
   . le reste de la couche étant constitué de groupements hydro- ou fluorocarbonés ;
(B) des surfaces obtenues par dépôt ou ancrage sur un solide d'une ou plusieurs couches moléculaires, celles-ci peuvent être obtenues par la formation de couches successives fixées par liaisons non-covalentes, type film de Langmuir-Blodgett, ou par auto-assemblage moléculaire, ceci permettant la formation d'une couche fixée par liaison covalente.

Dans le premier cas, la surface peut être obtenue par polymérisation d'au moins un monomère générant en surface du polymère ledit groupement comportant une double liaison éthylénique, ou bien par dépolymérisation partielle de la surface d'un polymère pour générer ledit groupement, ou encore par dépôt de polymère.

Dans ce procédé, le polymère formé présente des liaisons vinyls tel un dérivé polyénique, notamment des surfaces de type caoutchouc synthétique, tel que le polybutadiène, le polyisoprène ou le caoutchouc naturel.

Dans le deuxième cas, la surface hautement spécifique pour réactions biologiques selon la présente invention comporte :
- sur un support, une couche sensiblement monomoléculaire et compacte d'un composé organique de structure allongée ayant au moins :
   . un groupement de fixation présentant une affinité pour le support, et
   . un groupement exposé comportant une double liaison éthylénique, n'ayant pas ou peu d'affinité pour ledit support et ledit groupement de fixation dans les conditions de fixation, mais présentant une affinité pour un type de molécule biologique.

Afin d'obtenir une couche essentiellement compacte, les différents composés organiques sont, de préférence, susceptibles de réagir entre eux en dehors du groupement exposé pour créer des liaisons transversales, on obtient ainsi une couche monomoléculaire "essentiellement" compacte grâce à laquelle le support devient pas ou peu accessible pour des réactions parasites.

De préférence, le composé organique présente un groupement de fixation à une extrémité et un groupement exposé à l'autre extrémité. Il est bien entendu possible de prévoir des modes de réalisation différents dans lesquels, par exemple, le groupement de fixation serait situé au milieu de la molécule, celui-ci disposant à chacune de ses extrémités d'un groupement exposé.

Les surfaces peuvent s'analyser selon :
a) le support,
b) la molécule ayant un groupement exposé et un groupement de fixation sur le support,
c) l'interaction entre le support et ladite molécule assurant la fixation.

La fixation peut être tout d'abord de type non-covalent, notamment de type hydrophile/hydrophile et hydrophobe/hydrophobe, comme dans les films de Langmuir-Blodgett (K.B. Blodgett, J. Am. Chem. Soc. 57, 1007 (1935) et US 5.102.798).

Dans ce cas, le groupement de fixation sera, soit hydrophile, soit hydrophobe, notamment des groupements alkyles ou halogénoalkyles tels que CH₃, CF₃, CHF₃, CH₂F.

La fixation peut être également de type covalent, le groupement de fixation va alors réagir chimiquement sur le support.

Certaines surfaces de structure approchante ont déjà été mentionnées dans le domaine électronique, notamment lorsque les fixations sont covalentes, L. Netzer et J. Sagiv, J. Am. Chem. Soc. 105, 674 (1983) et US-A-4 539 061.

L'homme de l'art dispose d'une très large gamme de groupements. A titre d'exemple non limitatif on citera les groupements de type alkoxyde de métal, tel silane, chlorure de silane, éthoxysilane, méthoxysilane.

Le groupement de fixation est évidemment choisi en fonction du support employé. Le support selon l'invention peut être constitué au moins en surface, par un polymère, un métal, un oxyde de métal, un élément semi conducteur ou un oxyde d'élément semi conducteur tel qu'un oxyde de silicium ou une de leur combinaison. On cite en particulier le verre et le silicium oxydé en surface.

Parmi les groupements de fixation il faut citer plus particulièrement les groupements de type alkoxyde de métal tel silane, chlorosilane, chlorosilane, silanol, méthoxysilane, éthoxysilane, silazane, phosphate, hydroxy, hydrazide, hydrazine, amine, amide, diazonium, pyridine, sulfate, sulfonique, carboxylique, boronique, halogène, halogénure d'acide, aldéhyde.

Tout particulièrement, comme groupement de fixation on préfèrera utiliser des groupements susceptibles de réagir transversalement avec un groupe équivalent, voisin, pour fournir les liaisons transversales, par exemple il s'agira de dérivés de type silane, notamment dichlorosilane, trichlorosilane, diméthoxysilane, triméthoxysilane, diéthoxysilane et triéthoxysilane.

Ces liaisons transversales peuvent aussi être effectuées à un point quelconque dans l'épaisseur de la monocouche. en la polymérisant à l'aide de groupements réactifs éventuellement présents sur la chaîne entre le site de fixation et le groupement exposé. Ainsi, les groupements diacétyléniques sont connus pour permettre une polymérisation uni- ou bi-dimensionnelle de la monocouche.

Le choix du groupement de fixation dépendra évidemment de la nature du support, les groupements de type silane sont bien adaptés pour la fixation covalente sur le verre et la silice.

De préférence, les chaînes reliant le groupement exposé au groupement de fixation sont des chaînes comportant au moins 1 atome de carbone, de préférence plus de 6 et en général de 3 à 30 atomes de carbone. Lorsqu'il y a formation d'un couplage latéral à l'intérieur même de la couche, que ce couplage soit ionique, de coordinance ou covalent, on obtient des couches hautement ordonnées obtenues par auto-assemblage, même si la surface initiale ne présente qu'un nombre réduit de sites d'ancrage actifs comparé au nombre de molécules obtenues dans une monocouche compacte.

On peut avantageusement utiliser, dans le cas du verre ou de la silice, les techniques connues de fonctionnalisation de surface utilisant des dérivés silanes, par exemple : Si-OH + Cl₃-Si-R-CH=CH₂ donne Si-O-Si-R-CH=CH₂, R consistant par exemple en (CH₂)₄. Une telle réaction est connue dans la littérature, avec utilisation de solvant ultra-purs. La réaction conduit à un tapis de molécules présentant leur extrémité C=C à la surface exposée à l'extérieur.

Dans le cadre de l'obtention de surface à très haute spécificité, la présente invention concerne aussi pour de telles réactions de greffage de molécule à double liaison C=C l'utilisation d'une phase gazeuse, permettant d'éviter l'utilisation de solvant.

Dans le cas de l'or, celui-ci étant éventuellement sous la forme d'une couche mince sur un substrat, les techniques connues de fonctionnalisation de surface utilisent des dérivés thiols, par exemple : Au + HS-R-CH=CH₂ donne Au-S-R-CH=CH₂, R consistant par exemple en (CH₂)₄. Une telle réaction est décrite en milieu liquide et conduit, de même que la réaction précédente trichlorosilane-silice, à un tapis de molécules présentant leur extrémité C=C à la surface exposée à l'extérieur.

Bien entendu la terminologie de "support" englobe aussi bien une surface unique telle qu'une lame, mais également des particules qu'il s'agisse de poudre de silice ou de billes de polymère, et aussi des formes quelconques telles que barre, fibre ou support structuré, lesquelles peuvent d'ailleurs être rendues magnétiques, fluorescentes ou colorées, comme cela est connu dans différentes technologies de dosage.

De préférence, le support sera choisi pour être pas ou peu fluorescent lorsque la détection sera effectuée par fluorescence.

Les surfaces obtenues selon les modes (A) ou (B) ci-dessus présentent une grande spécificité grâce à la présence de sites réactifs spécifiques provenant, soit des groupes exposés, soit de la molécule fixée.

En outre, les surfaces obtenues selon les modes (A) ou (B) présentent les caractéristiques inattendues et remarquables suivantes :
(i) un ancrage spécifique fortement pH dépendant de l'ADN par ses extrémités sans nécessité d'une fonctionnalisation particulière de la molécule, accompagné d'un très faible taux d'interactions non-spécifiques ;
(ii) la possibilité d'y ancrer des protéines et d'autres molécules d'intérêt biologique, sans modification chimique particulière ;
(iii) la possibilité de préparer des surfaces spécifiques vis à vis d'un antigène (par exemple digoxigénine) ou d'un ligand (par exemple biotine) ;
(iv) un très faible taux de fluorescence intrinsèque, lorsque cela est requis, un bruit de fond de fluorescence (d'une aire typique de 100 x 100 µm) plus faible que le signal de fluorescence d'une seule molécule à détecter ;
(v) la possibilité de détecter des molécules isolées avec un rapport S/N indépendant du nombre de molécules, qui est. possible grâce à différentes techniques à grand rapport S/N décrites plus bas et basées sur l'identification de la présence d'un marqueur macroscopique présentant une faible interaction non-spécifique avec la surface.

Les surfaces ainsi obtenues sont, de préférence, revêtues d'une molécule à activité biologique choisie parmi :
- les protéines,
- les acides nucléiques,
- les lipides,
- les polysaccharides et leurs dérivés.

Parmi les protéines, il faut citer les antigènes et les anticorps, les ligands, les récepteurs, mais également des produits de type avidine ou streptavidine ainsi que les dérivés de ces composés.

Parmi les ARN et les ADN, il faut également citer les dérivés α, β ainsi que les dérivés thio et les composés mixtes tels que les PNA.

On peut également fixer des composés mixtes tels que les glycopeptides et les lipopolysaccharides par exemple, ou bien d'autres éléments tels que virus, cellules notamment, ou composés chimiques tels que la biotine.

La fixation des molécules biologiques peut être covalente ou non-covalente, par exemple par adsorption, liaisons hydrogènes, interactions hydrophobes, ioniques, par exemple, auquel cas on pourra procéder avantageusement à un pontage ("cross-linking") entre les molécules greffées par les méthodes connues ("Chemistry of Protein Conjugation and Cross-linking", S.C. Wong, CRC Press (1991)) et ceci afin de renforcer leur cohésion.

Avec un groupement exposé comportant un radical -CH=CH₂ qui sera nommé ci-après "surface C=C" ou "surface à liaison éthylénique", un ancrage direct, en particulier de l'ADN ou des protéines, est possible. Dans le cadre de la présente invention, il a été démontré que ces surfaces présentent une réactivité très fortement pH dépendante. Cette particularité permet d'ancrer les acides nucléiques ou les protéines, notamment par leur(s) extrémité(s), en utilisant une zone de pH déterminée et souvent avec une vitesse de réaction qui peut être contrôlée par le pH.

Ainsi, pour l'ADN à pH 5,5, la réaction d'ancrage est totale en une heure (si pas limitée par la diffusion) et se produit par les extrémités. A pH 8, par contre, l'accrochage est très faible (vitesse de réaction de 5 à 6 ordres de grandeur plus faibles). Cet effet d'accrochage pH dépendant et spécifique des extrémités, présente une amélioration par rapport aux autres surfaces qui nécessitent une fonctionnalisation de l'ADN (biotine, DIG, NHS, ...) ou des réactifs spécifiques (carbodiimide, diméthyle pimélidate) qui réalisent une liaison peptidique ou phosphorimide entre -NH₂ et -COOH ou -POOH.

Les surfaces selon l'invention peuvent ancrer des protéines directement (protéine A, anti-DIG, anticorps, streptavidine, etc.). Il a été observé que (i) l'activité de la molécule peut être préservée et (ii) que la réactivité de la surface préparée (initialement C=C) est totalement occultée pour faire place à la seule réactivité de la molécule d'intérêt. Il est donc possible, à partir d'une réactivité initiale relativement large, de passer à une surface possédant une réactivité très hautement spécifique, par exemple celle de sites spécifiques sur une protéine.

En ancrant un anticorps spécifique sur la surface (par exemple anti-DIG), on crée une surface dont la réactivité est limitée à l'antigène (par exemple le groupement DIG). Ceci indique que les groupements chimiques initiaux ont tous été occultés par les anticorps ancrés.

On peut aussi ancrer sur les surfaces réactives (chimiquement ou biochimiquement) d'autres molécules à activité biologique, notamment des virus ou d'autres composants : membranes, récepteurs membranaires, polysaccharides, PNA, notamment.

Il est également possible de fixer le produit d'une réaction d'intérêt biologique (par exemple la PCR) sur les surfaces préparées.

La présente invention concerne également les surfaces obtenues par la mise en oeuvre des procédés selon la présente invention et tous les procédés mettant en oeuvre ce type de surface, qu'il s'agisse de procédés permettant la mise en évidence et/ou la quantification de molécules biologiques, mais également la séparation de certaines molécules biologiques, notamment un prélèvement par mise en oeuvre des techniques de couplage antigène/anticorps et/ou ADN, ADN/ARN.

La présente invention concerne également des procédés de préparation des surfaces hautement spécifiques pour réactions biologiques tels que décrits précédemment pour l'obtention des couches selon (A) et (B) et, en particulier, le procédé caractérisé en ce que :
- on fixe sur un support une couche sensiblement monomoléculaire et compacte d'un composé organique de structure allongée ayant au moins :
   . un groupement de fixation présentant une affinité pour le support, et
   . un groupement exposé comportant une double liaison éthylénique, n'ayant pas ou peu d'affinité pour ledit support et le groupement de fixation dans les conditions de fixation, mais présentant une affinité pour un type de molécule biologique.

La présente invention concerne également les applications des surfaces traitées à la détection de molécules isolées à l'aide de réactifs spécifiques et de méthodes de détection à rapport S/N indépendant du nombre de molécules détectées.

Ainsi de façon générale, la présente invention concerne un procédé de mise en évidence et/ou de dosage d'une molécule à activité biologique dans un échantillon, caractérisé en ce qu'on utilise une surface telle que décrite précédemment, sur laquelle se trouve fixée une molécule à activité biologique capable de reconnaître la molécule de l'échantillon, et en ce que la mise en évidence ou le dosage sont effectués grâce à un réactif fluorescent ou non détectant la présence de la molécule fixée.

Parmi les réactifs on distingue les réactifs fluorescents et les réactifs non-fluorescents.

Les réactifs fluorescents contiennent des molécules fluorescentes, choisies avec avantage pour être des molécules longues de taille supérieure à 0,1 µm et réagissant de manière spécifique directement ou indirectement avec les surfaces prétraitées. Par exemple, mais sans pour autant s'y limiter, une molécule d'ADN double brin teintée à l'aide de sondes fluorescentes (ethidium bromide, YOYO, nucléotides fluorescents, etc.) pouvant s'ancrer directement sur une suface C=C, ou par une modification de la molécule (DIG, biotine, etc.) sur une surface présentant des protéines complémentaires (anti-DIG, streptavidine, etc.).

Les réactifs non-fluorescents consistent, notamment, en des billes ancrées par l'intermédiaire d'une molécule fixée de manière spécifique directement ou indirectement à une surface prétraitée. Grâce au traitement des surfaces, ces billes présentent une faible interaction non-spécifique avec la surface. Par exemple, mais sans pour autant s'y limiter, des billes Dynal recouvertes de streptavidine et ancrées par l'intermédiaire d'un ADN biotynilé à une surface selon la présente invention, présentant des sites capables de réagir avec l'autre extrémité de la molécule d'ADN.

Selon que la molécule recherchée est détectée directement par fluorescence ou indirectement à l'aide des réactifs ci-dessus, on parlera de "détection directe" ou "par drapeau".

Afin de limiter les problèmes associés à des temps de réaction prohibitivement lents, on peut avantageusement réduire les temps de diffusion des réactifs vers la surface en utilisant de petits volumes de réaction. Par exemple, mais sans s'y limiter en conduisant la réaction dans un volume de quelques microlitres déterminé par l'espacement entre deux surfaces dont l'une est traitée pour présenter des sites réactifs selon la présente invention et l'autre est inerte ou traitée pour ne pas présenter de sites réactifs.

La détection du nombre de réactions spécifiques s'étant produites peut être réalisée sur un petit nombre de molécules (typiquement 1 à 1 000), par un test physique macroscopique faible bruit ne nécessitant ni microscope électronique ni radioactivité ni nécessairement la PCR.

Les procédés de détection sont susceptibles d'être mis en oeuvre par des personnes n'ayant qu'une expérience de laboratoire réduite.

Selon le réactif, deux mises en oeuvre de la présente invention (mode X et mode Y) sont utilisables pour la détection macroscopique faible bruit d'un petit nombre de réactions d'ancrage du réactif.

Dans la mise en oeuvre dite de mode X de la présente invention, un test du nombre de réactions spécifiques s'étant produites est obtenu directement par une technique de fluorescence, permettant pour certaines formes de la réalisation de la présente invention d'identifier individuellement le nombre de sites ayant réagi. Dans ce cas, la surface hautement spécifique est prise avantageusement pour présenter un taux très faible de fluorescence, notamment le support doit présenter une faible fluorescence.

Après ancrage du réactif fluorescent, la détection et le comptage du nombre éventuellement petit de réactions d'ancrage peut se faite avantageusement avec l'aide d'un microscope optique à fluorescence utilisant un objectif à grande ouverture numérique, permettant de repérer soit directement à l'oeil, soit après acquisition de signal, le nombre de molécules fluorescentes ancrées.

On peut avantageusement procéder à un balayage du champ d'observation pour explorer une plus grande surface que le seul champ fixe.

Dans la mise en oeuvre dite de mode Y de la présente invention, on détecte un réactif macroscopique de type bille (fluorescente, magnétique, colorée, par exemple).

Une telle technique est dérivée de Manning et al. en ce sens que la réaction est révélée par la présence ou l'absence de microbilles. Dans un mode de réalisation un nouveau procédé comprend :
(i) l'utilisation de billes à réactivité spécifique,
(ii) l'utilisation de billes de tailles non pas nanoscopiques mais se situant dans la gamme 0,1 µm-200 µm, décelables par une technique macroscopique, et
(iii) l'absence de réaction non-spécifique entre billes et surface due à l'utilisation du produit selon la présente invention.

Le nombre de ces billes macroscopiques caractérisant chacune une réaction d'ancrage est ensuite déterminé par une méthode physique macroscopique au rang desquelles, mais sans s'y limiter, on peut citer la diffusion de lumière sur les billes, la microscopie optique et la fluorescence des billes.

La spécificité de certaines réactions biologiques peut être limitée. Ainsi, dans le cadre de l'hybridation, les hybrides peuvent être imparfaits (réactions avec d'autres sites) tout en présentant un nombre réduit d'appariement et donc une qualité de liaison moindre. La présente invention couvre également l'utilisation possible d'une étape de test de la qualité des liaisons obtenues. Ce test permet de dissocier les produits appariés de façon non-spécifique faible, par adsorption, forces hybrophobes, liaisons hydrogènes imparfaites, hybridation imparfaite, notamment.

C'est pourquoi, l'invention concerne également dans un procédé de mise en évidence ou de dosage tel que décrit précédemment, un procédé où l'on soumet le produit de réaction entre la molécule à activité biologique et la molécule de l'échantillon à une contrainte afin de détruire les mauvais appariements avant la détection.

Ce procédé offre, outre la possibilité de détruire les couples misappariés, la possibilité d'orienter les produits du couplage, ce qui facilite les mesures ou les observations.

On peut ainsi appliquer aux surfaces, après fixation des éléments complémentaires, une contrainte qui peut être constituée par l'utilisation simple ou combinée de :
- centrifugation,
- gradient de champ magnétique appliqué aux réactifs non-fluorescents pris alors pour inclure des microbilles magnétisables ou magnétiques,
- agitation,
- écoulement liquide,
- passage de ménisque,
- électrophorèse
- variation de température, et/ou gradient de température.

On détermine alors par les techniques de détection faible bruit décrites ci-après le nombre de systèmes étant restés intègres ou s'étant détruits.

Il convient de remarquer que grâce aux surfaces selon la présente invention, il est possible d'orienter les molécules après leur fixation par au moins un point par passage du ménisque air/eau, notamment sur de l'ADN. Ainsi, on a remarqué que le passage du ménique air/eau sur de l'ADN en solution et ancré à la surface, résultait en une extension régulière des molécules ancrées. Elles se présentent alors à l'air libre sous forme de bâtonnets fluorescents allongés. Ces molécules allongées sont stables à l'air libre et peuvent être observées même après plusieurs semaines, sans présenter de dégradation apparente.

Ces observations remarquables et inattendues suggèrent une possibilité de compter le nombre de molécules d'ADN ancrées à la surface : d'une part, les surfaces étant très peu fluorescentes, le rapport signal/bruit (S/N) est bon, d'autre part, recherchant un objet très corrélé (forme de bâtonnets), il est très facile d'augmenter le rapport S/N. C'est-à-dire, ignorer les poussières, les inhomogénéités, qui ne présentent pas de corrélation spatiale particulière. Il faut noter qu'en solution, les molécules en pelote fluctuent thermiquement, ce qui entraîne des variations très importantes de leur signal de fluorescence recueilli, en général, avec une faible profondeur de champ et limite leur observation. La présente invention couvre aussi cette technique d'alignement et d'immobilisation qui permet donc l'observation de molécules isolées avec un très grand rapport S/N.

Il est remarquable que ce rapport est indépendant du nombre de réactions d'ancrage. Le rapport S/N posé par la détection d'une molécule est le même que pour 10 000. De plus cette technique d'étirement permet de discriminer aisément entre des molécules de longueurs variées.

On peut avantageusement procéder aux étapes suivantes pour améliorer encore le rapport S/N :
- La molécule étant immobile, on peut intégrer son signal de fluorescence.
- L'observation au microscope présente un champ réduit (typiquement 100 µm x 100 µm avec un objectif x 100 à immersion, N.A. = 1.25). Pour un échantillon de 1 cm² on peut soit procéder à un balayage, soit envisager l'utilisation d'objectifs d'agrandissements moindres (x 10 ou x 20) mais d'ouverture numérique élevée.
- Les bâtonnets étant toujours parallèles, on peut envisager une méthode de filtrage spatial optique pour augmenter encore le rapport S/N.
- D'autres méthodes de fluorescence globale sont envisageables (EP # 103426).
- La linéarisation des molécules s'observe aussi bien dans le cadre d'un greffage chimique (C=C) que dans le cas de liaisons de type immunologique (DIG/anti-DIG).
- Une fois la surface à l'air libre, les molécules d'ADN sont stables (restent intègres, même après plusieurs semaines) et fluorescentes. On peut avantageusement utiliser cette propriété pour différer l'étape d'ancrage de l'étape de repérage/comptage des molécules ancrées, si cette détection se fait par exemple, mais sans s'y limiter, par microscopie à fluorescence. Une telle utilisation est couverte par la présente invention.
- Une technique de double (ou multi) fluorescence peut éventuellement servir à améliorer le rapport S/N ou à détecter une double ou multi fonctionnalité.
- Il est possible d'étendre le ménisque air/eau utilisé ici afin d'étirer la molécule à d'autres systèmes tels que huile/eau ou eau/surfactant/air, notamment.

On peut aussi utiliser une orientation dynamique des molécules en solution ancrées à une extrémité, par électrophorèse ou écoulement dans une ou plusieurs directions successives, une telle technique pouvant ainsi conduire à une détection synchrone de la présence de molécules dans une direction donnée, par analyse des variations temporelles du signal de fluorescence correspondant à une direction donnée (par exemple, mais sans sy limiter pour autant, par utilisation d'un filtre spatial optique convenablement disposé, permettant d'obtenir un signal préférentiel pour certaines orientations des molécules observées).

Néanmoins, les résultats observés montrent que cette technique, dans sa version la plus simple (étirement dans une seule direction, sans détection synchrone) est beaucoup moins performante que l'utilisation du ménisque.

Les surfaces et/ou les réactifs et/ou les techniques de détection décrits dans la présente invention peuvent être utilisés pour de nombreuses applications parmi lesquelles, mais sans s'y restreindre :
- l'identification d'un ou plusieurs éléments de séquençage d'ADN ou d'ARN que l'on peut utiliser avec avantage pour le diagnostic de pathogènes ou la cartographie génétique ;
- la mesure de la taille de fragments d'ADN que l'on peut utiliser avec avantage pour la cartographie génétique ;
- l'amélioration de la sensibilité des techniques d'ELISA avec la possibilité de détecter un faible nombre (éventuellement inférieur à 1 000) de réactions immunologiques.

L'identification de séquences d'ADN/ARN peut se faire d'abord par réaction dans le volume de la solution des molécules d'ADN/ARN avec des sondes complémentaires (par exemple par hybridation ou à l'aide de protéines spécifiques du segment recherché). Deux modes d'opération sont alors possibles.

Les descriptions qui vont suivre seront, pour certaines, faites en se référant aux figures annexées sur lesquelles :
- la figure 1 schématise la détection d'un pathogène dans une molécule d'ADN fluorescente par hybridation avec une molécule ancre ;
- la figure 2 schématise la cartographie génétique par extension de l'ADN et l'utilisation d'un ADN marqueur ;
- la figure 3 schématise la détection d'une réaction immunologique (ELISA) à l'aide d'une molécule "drapeau" : un ADN fluorescent utilisé comme marqueur de réaction ;

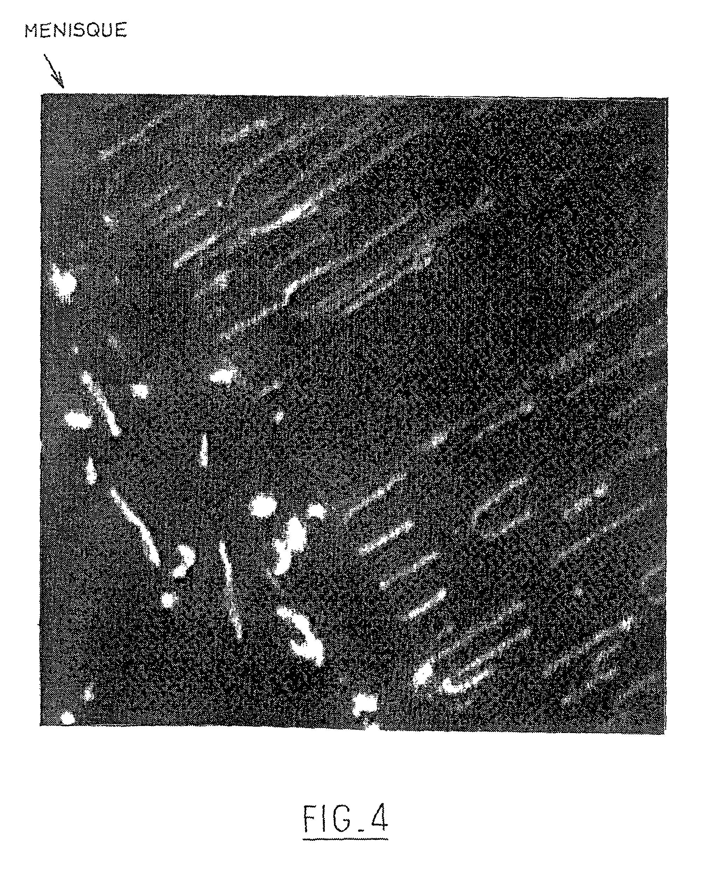

- la figure 4 est une microphotographie de fluorescence montrant l'extension d'ADN de phage λ par l'avancée du ménisque, à gauche on aperçoit des molécules d'ADN en solution étirées par l'écoulement d'évaporation parallèle au ménisque, à droite des molécules d'ADN à l'air libre après leur étirement perpendiculairement au ménisque ;

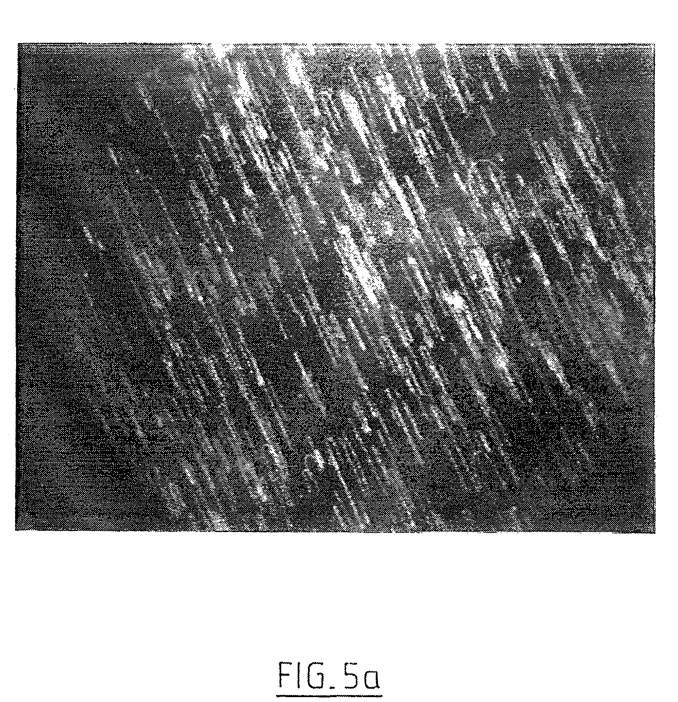

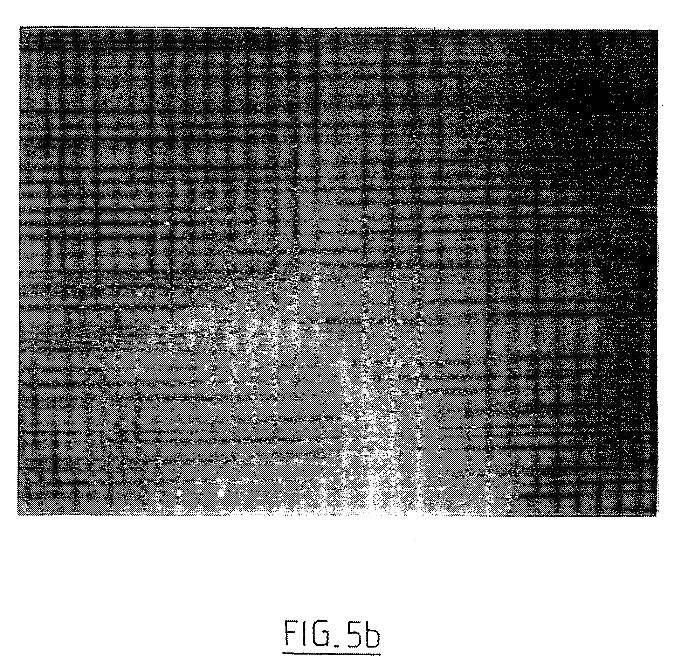

- les figures 5(a) et 5(b) sont des microphotographies de fluorescence montrant, respectivement, un ADN marqué à la digogixénine (DIG) sur une surface recouverte d'anti-DIG et étiré par le ménisque, et, en contrôle, un ADN non marqué sur une surface anti-DIG, on remarquera la très grande spécificité des surfaces et l'absence d'ancrage non-spécifique ;

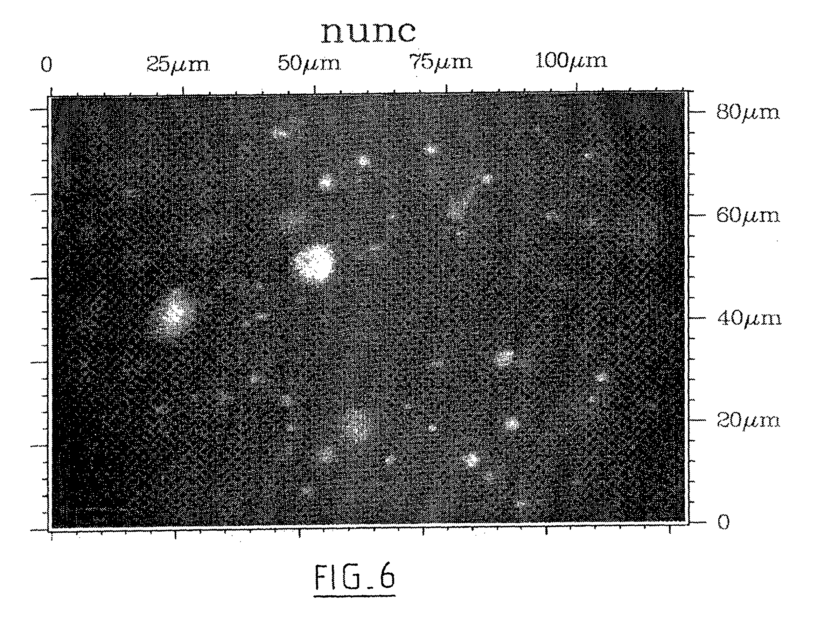

- la figure 6 est une microphotographie de fluorescence montrant une surface commerciale classique telle que NUNC, on remarquera les très grandes inhomogénités de fluorescence qui rendent ces surfaces impossibles à utiliser pour la détection fluorescente d'une seule molécule.

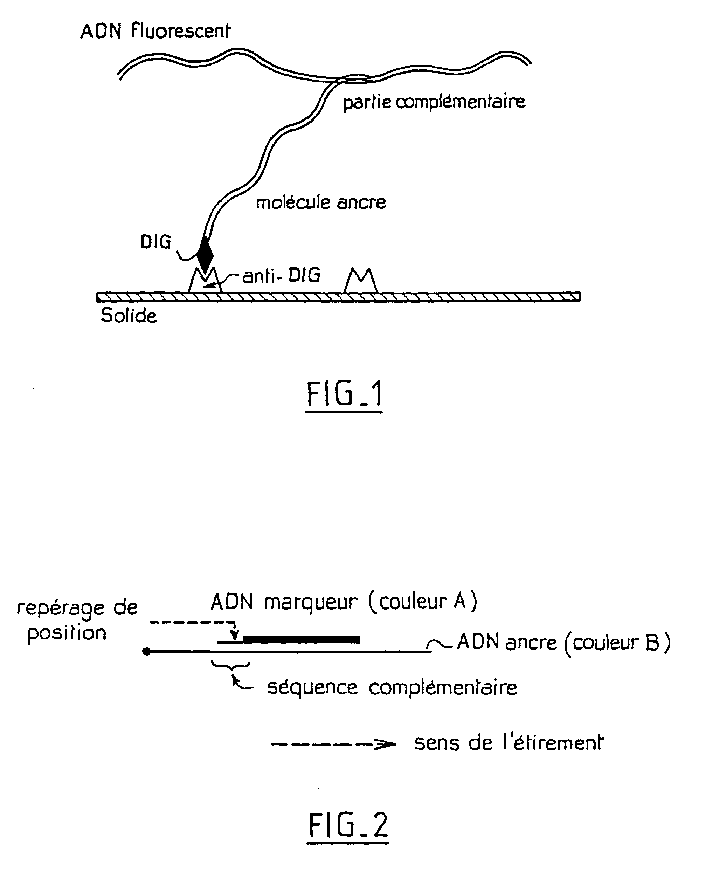

Dans le mode "diagnostic", les sondes (les "ancres") possèdent un groupement réactif (DIG, biotine, etc.) capable de s'ancrer de manière spécifique à une surface selon la présente invention (ayant par exemple comme site d'ancrage un anticorps anti-DIG ou la streptavidine). La détection de la réaction d'ancrage peut se faire directement par détection de la fluorescence de la molécule d'ADN teintée par des molécules fluorescentes (ethidium bromide, YOYO, nucléotides fluorescents) (figure 1). Elle peut aussi se faire indirectement par détection d'une "molécule drapeau" : un réactif selon la présente invention capable de se fixer sur la molécule d'ADN/ARN (par exemple par hybridation, interaction protéine-ADN, etc.), mais ne présentant pas d'affinité pour les sites d'ancrage de la sonde.

Dans le mode "cartographie", les sondes complémentaires peuvent être directement couplées à un réactif fluorescent selon la présente invention. Il peut s'agir par exemple d'un simple brin d'ADN complémentaire possédant des bases modifiées pour être fluorescentes ou d'un long double brin d'ADN teinté avec un fluorophore A et se terminant par un segment simple brin complémentaire de la séquence recherchée. Pour des sondes différentes, des fluorophores de différentes couleurs peuvent être utilisés. On peut aussi, avec avantage, teindre la molécule d'ADN sur laquelle les sondes viennent s'hybrider avec un fluorophore d'une autre couleur. L'hybride ADN-sondes est ancré en l'une de ses extrémités et étiré par une des méthodes décrites précédemment. La distance du point d'ancrage aux points d'hybridation,ou entre les points d'hybridations, est déterminée par la détection de la fluorescence de la sonde, suivant les méthodes décrites précédemment (figure 2).

Par exemple, sans pour autant s'y limiter, un ADN marqueur, d'environ 3 000 paires de bases et ayant en une de ses extrémités un segment simple brin complémentaire du gène recherché, est teinté avec un fluorophore A (par exemple YOYO1). Cet ADN est hybridé puis ligué avec l'ADN simple brin à cartographier, puis ce dernier est teinté avec un deuxième fluorophore B (POPO1) (après réaction par "random primming", pour le transformer en ADN double brin). La molécule est alors ancrée par une de ses extrémités (par exemple par liaison DIG/anti-DIG) et étirée par l'action du ménisque. La distance entre l'extrémité de la molécule et la postion du gène marqué, observable en microscopie par double fluorescence (2 couleurs A et B) permet d'établir la position du gène recherché avec une précision de l'ordre de 1 000 paires de bases (0,3 µm).

L'identification de séquences d'ADN/ARN peut aussi se faire par réaction entre la séquence recherchée et les sites réactifs d'une surface selon la présente invention (par exemple des oligonucléotides complémentaires ou le site de réaction d'une protéine spécifique du segment recherché). La détection de la réaction d'ancrage peut alors se faire directement ou indirectement (à l'aide d'une "molécule drapeau") comme décrit précédemment.

Il est bien entendu que l'identification de séquences d'ADN/ARN suivant la présente invention peut aussi bien servir à des fins de diagnostic (par exemple la détection de la présence ou l'absence d'un pathogène viral ou chromosomique) qu'à des fins de cartographie génétique. Elle peut être précédée d'une étape d'amplification par une méthode quelconque, notamment la PCR.

Par ailleurs, comme mentionné par K.R. Allan et al. (US 84 114), La cartographie génétique peut procéder par une mesure de la taille de fragments d'ADN. Or le couplage entre les surfaces selon la présente invention et les techniques originales d'étirement des molécules décrites plus haut (en particulier et avec avantage l'étirement par le ménisque) permet une mesure de la longueur des molécules étirées et cela sur un très petit échantillon (quelques milliers de molécules).

On peut, par exemple, mais sans sy restreindre, procéder de la manière suivante :

Un échantillon d'ADN est fragmenté (à l'aide d'enzymes de restriction), teinté avec un fluorophore puis ancré sur une surface présentant des groupements réactifs (par exemple les surfaces C=C). Les molécules sont ensuite étirées par le ménisque et la taille des fragments étirés déterminée par microscopie optique à fluorescence avec une résolution et une taille limite de l'ordre de 1 000 bp (0,3 µm).

Les surfaces selon la présente invention peuvent être utilisées pour la mise en oeuvre des procédés connus permettant la mise en évidence et/ou la quantification d'un antigène ou d'un anticorps, notamment les méthodes ELISA mettant en oeuvre des systèmes enzymatiques ou des méthodes de type RIA mettant en oeuvre des marqueurs radioactifs. Il s'agit là de technologies qui ne seront pas redécrites en détail.

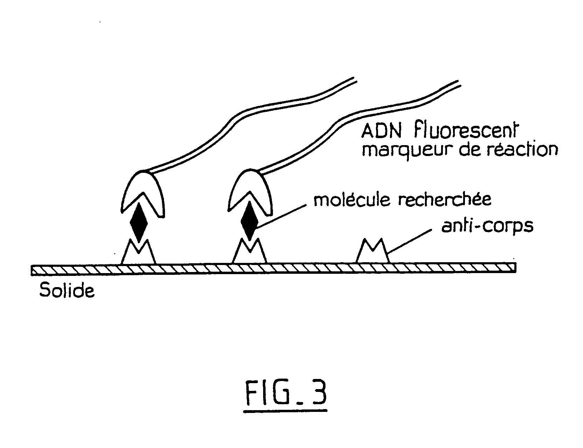

On peut aussi et avantageusement utiliser les surfaces selon la présente invention comme support des réactions immunologiques d'un procédé ELISA possédant une étape d'ancrage d'un réactif selon la présente invention ("drapeau") sur l'un des réactifs de l'ELISA (figure 3). La détection peut naturellement se faire de façon globale par mesure de fluorescence. Il est possible aussi de procéder au comptage du nombre de réactions, ceci peut avantageusement s'effectuer suivant les méthodes de détection décrites dans la présente invention, en particulier l'extension par le ménisque, et ceci grâce au faible taux de fluorescence et d'interaction non-spécifique du produit de la présente inention. Ceci permet la détection d'un petit nombre de réaction, éventuellement inférieur à 1 000) avec un excellent rapport S/N.

On peut donc, par une modification mineure des méthodes ELISA en sandwich (anticorps-antigène-anticorps modifié, par exemple biotynilé), venir greffer sur la surface un réactif selon la présente invention, par exemple de l'ADN fluorescent ancré à la streptavidine.

Toutes les variantes de la technique ELISA s'appliquent avec une sensibilité bien meilleure. Des techniques de mesure de fluorescence globale sont déjà utilisées pour déterminer la qualité des réactions d'ELISA. Cependant, le procédé selon l'invention permet une détection bien meilleure car sensible au signal de fluorescence d'une seule molécule.

Bien entendu, il est possible d'utiliser ces surfaces comme surfaces de fixation d'au moins un produit d'une réaction d'intérêt biologique, à titre d'exemple sans pour autant s'y limiter, des produits de la réaction d'amplification, qu'il s'agisse de PCR ou d'une méthode apparentée et, enfin, il est possible d'utiliser ce type de surfaces comme support pour une chromatographie d'affinité, quelle soit préparative ou de détection.

Dans ce cas, on peut par exemple greffer une population d'oligonucléotides donnée sur des billes de silice qui constitueront la phase stationnaire d'une colonne de chromatographie.

L'étape de chromatographie doit permettre une spécificité particulière de la colonne vis à vis d'éluants, par exemple un mélange d'ADN dont certains présentent des séquences complémentaires ou très voisines de l'oligonucléotide greffé.

La présente invention concerne enfin l'utilisation des surfaces selon la présente invention dans des trousses de diagnostic ou de séparation.

D'autres caractéristiques et avantages de la présente invention apparaîtront à la lecture des exemples ci-après.

### Matériels et méthodes

L'ADN-λ et l'anticorps monoclonal (Anti-DIG) proviennent de Boehringer-Mannheim. Les trichlorosilanes proviennent de Roth-Sochiel. Les sondes nucléiques fluorescentes (YOYO1, YOYO3 et POPO1) proviennent de Molecular Probes. Les lamelles de verre ultrapropres proviennent de Erie Scientific (Lamelles (ESCO). Les particules magnétiques proviennent de Dynal. Le microscope est un microscope inversé Diaphot de NIKKON, équipé d'une lampe Xenon pour l'épifluorescence et d'une caméra CCD intensifiée Hamamatsu pour la visualisation.

### Traitement de surface

Des lamelles de verre sont nettoyées pendant une heure par irradiation UV sous atmosphère d'oxygène (par formation d'ozone). Elles sont ensuite immédiatement déposées dans un dessicateur préalablement purgé de traces d'eau par un courant d'argon. Un volume d'environ 100 à 500 µl du trichlorosilane approprié (H₂C=CH-(CH₂)_{N}-SiCl₃ est introduit dans le dessicateur, d'où les surfaces sont retirées après environ 12 heures (*n* = 6) ou 1 heure (*n* = 1). Au sortir les surfaces sont nettes et non-mouillantes.

Les lamelles ainsi fonctionnalisées peuvent réagir avec des protéines. Un volume de 300 µl d'une solution aqueuse (20 µg/ml) de protéines (protéine A, streptavidine, etc.) est déposé sur une lamelle fonctionnalisée en groupement (H₂C=CH-). Cette lamelle est incubée environ deux heures à température ambiante, puis rincée trois fois dans de l'eau ultrapure. Les surfaces ainsi traitées sont nettes et mouillantes. Les surfaces traitées à la protéine A peuvent ensuite réagir avec un anticorps, par exemple anti-DIG, par incubation dans une solution de 20 µg/ml d'anticorps.

### Ancrage d'ADN natif sur surface double liaison

Une goutte de 2 µl d'une solution d'ADN-λ marqué par fluorescence (YOYO1, POPO1 ou YOYO3, mais sans terminaison particulière) de concentration variable et dans différents tampons (nombre total de molécules < 10⁷) est déposé sur une lamelle prétraitée (H₂C=CH-) et recouverte d'une lamelle de verre non traitée (diamètre 15 mm). La préparation est incubée environ 1 heure à température ambiante dans une atmosphère saturée en vapeur d'eau. Dans un tampon de 0,05 M MES (pH = 5,5), on observe un ancrage quasi-général des molécules d'ADN. Par contre dans un tampon de 0,01 M Tris (pH = 8) il n'y a presqu'aucune molécule d'ancrée (rapport > 10⁶. Cette dépendance peut permettre le contrôle de l'activation/désactivation des surfaces (vis-à-vis de l'ADN) par l'intermédiaire du pH.

### Détection de l'ancrage par l'action du ménisque

En transférant la préparation précédente dans une atmosphère sèche, la solution en s'évaporant va étirer les molécules d'ADN, ancrées à la surface, perpendiculairement au ménisque. La force capillaire sur la molécule d'ADN (quelques dizaines de picoNewtons) est en effet suffisante pour étirer complètement la molécule (plus grande que les forces d'élasticité entropique), mais trop faible pour briser la liaison entre l'extrémité de la molécule et la surface traitée. L'ADN étant marqué par fluorescence, on observe individuellement et aisément les molécules étirées (longueur totale environ 22 µm). L'ancrage entre la surface et l'ADN étant limité aux extrémités, on a pu aussi bien étirer des ADN de phage λ, de YAC ou de E. coli (longueur totale supérieure à 400 µm). Cette préparation d'ADN étirés, fluorescents et à l'air libre est stable pendant plusieurs jours et peut être observée de façon non destructive, par épifluorescence (microscope inversé Nikkon Diaphot avec objectif x100, O.N.: 1.25).

### Détection de l'ancrage par électrophorèse

Une cellule électrophorétique est formée par un anneau de paraffine (épaisseur environ 100 µm) pris entre une lamelle traitée et une lamelle de verre non-traitée entre lesquelles deux électrodes de platine sont insérées. L'ensemble est rendu solidaire en fondant brièvement l'anneau de paraffine. Grâce à deux ouvertures laissées dans l'anneau de paraffine on introduit la solution d'ADN dans cette cellule par capillarité, puis on scelle les deux ouvertures à la paraffine. On incube, comme précédemment, à température ambiante. En appliquant une faible tension (quelques volts) entre les deux électrodes de platine, on observe par fluorescence un mouvement des molécules d'ADN libre(quelques dizaines de microns par seconde) et une extension dans la direction de l'écoulement des molécules ancrées que l'on peut ainsi identifier aisément et individuellement par microscopie en épifluorescence.

### Ancrage et détection spécifiques

En traitant les surfaces comme décrit précédemment avec un anticorps monoclonal spécifique, on peut contrôler très précisément leur spécificité. Ainsi, on a testé la spécificité de surfaces traitées anti-DIG vis-à-vis d'ADN-λ hybridés avec un oligonucléotide complémentaire d'une des extrémités Cos et possédant un groupement digoxigénine (DIG) et vis-à-vis d'ADN non hybridés. Dans le premier cas, on a observé une extension, par action du ménisque, quasi-générale des molécules ancrées. Dans le second cas, on n'a observé que quelques molécules d'ADN (< 10) ancrées dans tout l'échantillon. On estime donc que la spécificité de la méthode selon l'invention est meilleure que 10⁶.

### Sensibilité de la détection

Afin de déterminer la sensibilité de la méthode de détection par extension du ménisque, on a déposé sur des surfaces double liaison des gouttes de 2,5 µl d'une solution d'ADN-λ dans 0,05 M MES (pH = 5,5) contenant un total de 10⁵, 10⁴ et 1 000 molécules. L'ancrage s'effectue comme décrit précédemment. Les lamelles sont ensuite observées en microscopie par épifluorescence, pour déterminer la densité de molécules ancrées. Celle-ci correspond bien à celle estimée : environ 4-6 molécules d'ADN par champ de vision (100 µm x 100 µm) pour un total de 10⁵ molécules d'ADN. Pour la plus faible concentration, on a pu observé une dizaine de molécules étendues par action du ménisque. Ce nombre est essentiellement limité par le grand nombre de champs de vision nécessaires à couvrir tout l'échantillon (environ 25 000), ce qui rend une recherche manuelle difficile, mais peut être avantageusement effectuée automatiquement et avec un objectif plus faible, mais à plus grand champ. En conclusion, la sensibilité de la méthode selon l'invention permet une détection et un comptage individuel de moins de 1 000 molécules d'ADN.

## Revendications

1. Surface hautement spécifique pour réactions biologiques, **caractérisée en ce qu'**elle comporte un support présentant en surface au moins une couche essentiellement compacte d'un composé organique présentant. à l'extérieur de la couche, un groupement exposé comprenant une double liaison éthylénique ayant une affinité pour un type de molécule à activité biologique dans certaines conditions de pH ou de teneur ionique, les autres éléments de la couche étant essentiellement inaccessibles pour lesdites molécules dans lesdites conditions de réaction.

2. Surface selon la revendication 1, **caractérisée en ce que** le support est un support solide.

3. Surface hautement spécifique selon l'une des revendications 1 ou 2, **caractérisée en ce que** le groupement exposé est un groupement vinyl.

4. Surface selon l'une des revendications 1 à 3, **caractérisée en ce qu'**elle est obtenue par polymérisation d'au moins un monomère générant en surface du polymère à double liaison éthylénique ledit groupement exposé, par dépôt de polymère sur le support ou par dépolymérisation partielle de la surface d'un polymère pour générer ledit groupement exposé.

5. Surface selon la revendication 4, **caractérisée en ce que** le polymère est une polyoléfine ou un dérivé polyénique.

6. Surface hautement spécifique selon l'une des revendications 1 à 5, **caractérisée en ce qu'**elle comporte :
- sur un support une couche sensiblement monomoléculaire et compacte d'un composé organique de structure allongée ayant au moins :
. un groupement de fixation présentant une affinité pour le support, et
. un groupement exposé comprenant une double liaison éthylénique n'ayant pas ou peu d'affinité pour ledit support et ledit groupement de fixation dans les conditions de fixation, mais présentant une affinité pour un type de molécule biologique.

7. Surface selon la revendication 6, **caractérisée en ce que** la fixation est de type non-covalente.

8. Surface selon la revendication 7, **caractérisée en ce que** la fixation est effectuée par des interactions hydrophiles ou hydrophobes.

9. Surface selon la revendication 8, **caractérisée en ce que** la molécule biologique est capable de s'ancrer directement par adsorption sur ladite surface dans certaines conditions de pH ou de teneur ionique du milieu.

10. Surface selon l'une des revendications 6 à 9, **caractérisée en ce que** le groupement de fixation est choisi parmi les groupements alkyle et alkyle halogéné pour réaliser des liaisons hydrophobes.

11. Surface selon la revendication 10, **caractérisée en ce que** le groupement de fixation est choisi parmi : -CH₃, CF₃, -CH₂F, CHF₂.

12. Surface selon la revendication 6, **caractérisée en ce que** la fixation est de type covalent.

13. Surface selon l'une des revendications 1 à 12, **caractérisée en ce que** les composés organiques sont susceptibles de réagir entre eux en dehors du groupement exposé pour créer des liaisons transversales.

14. Surface selon l'une des revendications 6 à 13, **caractérisée en ce que** le composé organique présente un groupement de fixation à une extrémité et un groupement exposé à l'autre extrémité.

15. Surface selon l'une des revendications 6 à 14, **caractérisée en ce que** le groupement de fixation est choisi parmi les groupements de type alkoxyde de métal, tel silane, chlorosilane, méthoxysilane et éthoxysilane, silanol, silazane, de type phosphate, hydroxy, hydrazide, hydrazine, amine, amide, diazonium, pyridine, sulfate, sulfonique, carboxylique, boronique, halogène, halogénure d'acide et aldéhyde.

16. Surface selon la revendication 15 **caractérisée en ce que** le groupement de fixation est choisi parmi les silanes tels que le mono, di ou tri-chlorosilane, mono, di ou tri-éthoxysilanes et mono, di ou tri-méthoxysilanes.

17. Surface selon l'une des revendications 1 à 16 **caractérisée en ce que** le groupement exposé est soit relié directement au groupement de fixation, soit par des chaînes comportant au moins un atome de carbone et de préférence de 3 à 30 atomes.

18. Surface selon l'une des revendications 1 à 17 **caractérisée en ce que** le support est constitué au moins en surface par un polymère, un métal ou un oxyde de métal, un élément semi-conducteur, un oxyde d'élément semi-conducteur, notamment un oxyde de silicium, ou une de leur combinaison.

19. Surface selon l'une des revendications 1 à 18, **caractérisée en ce que** le support est constitué au moins en surface par du verre ou de la silice.

20. Surface selon l'une des revendications 1 à 19, **caractérisée en ce que** le support est sous forme de plaque de bille, de fibre, ou de particules.

21. Surface selon la revendication 20, **caractérisée en ce que** lesdites particules sont magnétiques, fluorescentes ou colorées.

22. Surface selon l'une des revendications 1 à 20, **caractérisée en ce que** la molécule à activité biologique est choisie parmi :
- les protéines,
- les acides nucléiques,
- les lipides,
- les polysaccharides et leurs dérivés.

23. Surface selon l'une des revendications 1 à 22, **caractérisée en ce que** la molécule à activité biologique est un composé chimique présentant une activité biologique.

24. Surface selon l'une des revendications 1 à 23, **caractérisée en ce que** la molécule à activité biologique est choisie parmi :
- les protéines,
- l'ADN,
- l'ARN et leurs dérivés.

25. Surface selon la revendication 24, **caractérisée en ce que** la molécule à activité biologique est choisie parmi ;
- les anticorps,
- les antigènes,
- les ADN et ARN;
- les ligands ou leurs récepteurs ainsi que leurs dérivés.

26. Procédé de préparation d'une surface hautement spécifique pour réactions biologiques selon l'une des revendications 1 à 25, **caractérisé en ce que** :
- on fixe sur un support solide une couche sensiblement monomoléculaire et essentiellement compacte d'un composé organique de structure allongée ayant au moins :
. un groupement de fixation présentant une affinité pour le support, et
. un groupement exposé n'ayant pas ou peu d'affinité pour ledit support et le groupement de fixation dans les conditions de fixation, mais présentant une affinité pour un type de molécule à activité biologique.

27. Surface revêtue d'une molécule à activité biologique, **caractérisée en ce qu'**elle comporte une surface hautement spécifique pour réactions biologiques selon l'une des revendications 1 à 25 sur laquelle est fixée ladite molécule à activité biologique.

28. Surface selon la revendication 27, **caractérisée en ce que** la molécule à activité biologique est choisie parmi :
- les protéines,
- les acides nucléiques,
- les lipides,
- les polysaccharides et leurs dérivés.

29. Surface selon la revendication 28, **caractérisée en ce que** l'ADN fixé comporte la séquence complémentaire d'une séquence d'ADN à isoler d'un échantillon.

30. Surface selon la revendication 28, **caractérisée en ce que** la protéine fixée est capable de reconnaître et fixer spécifiquement une protéine à isoler d'un échantillon.

31. Surface selon l'une des revendications 28 et 29, **caractérisée en ce que** ladite molécule à activité biologique est choisie parmi la biotine, l'avidine, la streptavidine ou leurs dérivés.

32. Procédé d'identification de la présence d'un ADN ou d'un ARN mettant en oeuvre une surface selon l'une des revendications 1 à 31.

33. Procédé d'identification de la présence d'une protéine ou d'un anticorps mettant en oeuvre une surface selon l'une des revendications 1 à 31.

34. Procédé de mise en évidence et/ou de dosage d'une molécule à activité biologique dans un échantillon, **caractérisé en ce qu'**on utilise une surface selon l'une des revendications 27 à 31 sur laquelle se trouve fixée une molécule à activité biologique capable de reconnaître la molécule de l'échantillon, et **en ce que** la mise en évidence ou le dosage sont effectués grâce à un réactif fluorescent ou non détectant la présence de la molécule fixée.

35. Procédé selon la revendication 34, **caractérisée en ce que** la surface est à faible fluorescence et **en ce que** le réactif est fluorescent.

36. Procédé selon la revendication 35, **caractérisée en ce que** le réactif est constitué par des billes.

37. Procédé selon l'une des revendications 34 à 36, **caractérisé en ce que** la détection est faite par microscopie.

38. Procédé selon l'une des revendications 34 à 37, **caractérisé en ce qu'**on soumet le produit de réaction entre la molécule à activité biologique et la molécule de l'échantillon à une contrainte afin de détruire les mauvais appariements avant la détection.

39. Procédé de mise en évidence d'une séquence d'ADN ou d'une protéine dans un échantillon, **caractérisé en ce que** :
- on utilise une surface hautement spécifique selon l'une des revendications 1 à 31 :
- on met l'échantillon en contact avec la surface dans des conditions de formation de l'hybride ADN/ADN, ADN/ARN ou protéine/protéine.
- l'hybride étant marqué, on l'étire par tout moyen physico-chimique pour orienter les molécules et on effectue la mesure ou l'observation des molécules ainsi orientées.

40. Procédé selon l'une des revendications 38 et 39, **caractérisé en ce qu'**on étire l'hybride ou produit de réaction par passage d'un ménisque dans le sens de l'étirement.

41. Procédé selon la revendication 40, **caractérisé en ce que** le ménisque est un ménisque air/eau.

42. Procédé selon l'une des revendications 38 et 39, **caractérisé en ce que** l'étirement est effectué par un champ électrique agissant sur les molécules ou un champ magnétique agissant sur une particule magnétisable ou magnétique.

43. Procédé selon l'une des revendications 38 et 39, **caractérisé en ce que** l'étirement est effectué par voie mécanique.

44. Procédé selon l'une des revendications 38 à 43, **caractérisé en ce que** l'ADN fixé et l'ADN de l'échantillon sont "colorés" de façon différente et **en ce que**, après étirement, on mesure la position de la séquence complémentaire par rapport à l'extrémité de l'ADN de l'échantillon.

45. Procédé selon la revendication 44 destiné à la cartographie des gènes.

46. Procédé selon l'une des revendications 44 à 45, **caractérisé en ce qu'**il s'agit d'une méthode EUSA ou RIA.

47. Procédé selon l'une des revendications 44 à 45, **caractérisé en ce que** l'échantillon est le produit d'une amplification d'acide nucléique.

48. Procédé de fixation d'un acide nucléique ou d'une protéine sur une surface telle que définie selon l'une des revendications 1 à 25, dans lequel on réalise un ancrage par adsorption de l'ADN ou de la protéine notamment par une extrémité en mettant l'ADN ou la protéine en présence de la surface dans une zone de pH déterminée.

49. Procédé de fixation de l'ADN sur une surface présentent des groupements à double liaison éthylénique selon la revendication 48, **caractérisé en ce qu'**on met l'ADN en présence de la surface à un pH inférieur à 8.

50. Procédé de fixation selon la revendication 49, **caractérisé en ce que** la réaction est conduite à un pH compris entre 5 et 6 puis est stoppée à pH 8.

51. Trousse de diagnostic, **caractérisée en ce qu'**elle comprend au moins une surface hautement spécifique selon l'une des revendications 1 à 33.

52. Trousse de diagnostic selon la revendication 51, **caractérisée en ce qu'**elle comporte, en outre, un réactif fluorescent ou un réactif comportant des billes.

## Patentansprüche

1. Hochspezifische Oberfläche für biologische Reaktionen, **dadurch gekennzeichnet, dass** sie einen Träger umfasst, welcher an der Oberfläche wenigstens eine im wesentlichen kompakte Schicht von einer organischen Verbindung aufweist, welche auf der Außenseite der Schicht eine exponierte Gruppierung, welche eine ethylenische Doppelbindung umfasst, mit einer Affinität für einen Molekültyp mit biologischer Aktivität unter bestimmten pH- oder Ionengehalt-Bedingungen aufweist, wobei die anderen Bestandteile der Schicht für die genannten Moleküle unter den genannten Reaktionsbedingungen im wesentlichen unzugänglich sind.

2. Oberfläche nach Anspruch 1, **dadurch gekennzeichnet, dass** der Träger ein fester Träger ist.

3. Hochspezifische Oberfläche nach einem der Ansprüche 1 oder 2, **dadurch gekennzeichnet, dass** die exponierte Gruppierung eine Vinylgruppierung ist.

4. Oberfläche nach einem der Ansprüche 1 bis 3, **dadurch gekennzeichnet, dass** sie erhalten wird durch Polymerisation von wenigstens einem Monomer, welche an der Oberfläche des Polymers mit ethylenischen Doppelbindungen die exponierte Gruppierung erzeugt, durch Abscheidung von Polymer auf dem Träger oder durch partielle Depolymerisation der Oberfläche eines Polymers, um die exponierte Gruppierung zu erzeugen.

5. Oberfläche nach Anspruch 4, **dadurch gekennzeichnet, dass** das Polymer ein Polyolefin oder ein Polyen-Derivat ist.

6. Hochspezifische Oberfläche nach einem der Ansprüche 1 bis 5, **dadurch gekennzeichnet, dass** sie umfasst:
- auf einem Träger eine im wesentlichen monomolekulare und kompakte Schicht von einer organischen Verbindung von langgestreckter Struktur, die wenigstens aufweist:
. eine Anheftungsgruppe, die eine Affinität für den Träger aufweist, und
. eine exponierte Gruppierung, die eine ethylenische Doppelbindung umfasst, die keine oder nur geringe Affinität für den Träger und die Anheftungsgruppe unter den Anheftungsbedingungen aufweist, aber eine Affinität für einen biologischen Molekültyp aufweist.

7. Oberfläche nach Anspruch 6, **dadurch gekennzeichnet, dass** die Anheftung vom nicht-kovalenten Typ ist.

8. Oberfläche nach Anspruch 7, **dadurch gekennzeichnet, dass** die Anheftung durch hydrophile oder hydrophobe Wechselwirkungen bewirkt wird.

9. Oberfläche nach Anspruch 8, **dadurch gekennzeichnet, dass** das biologische Molekül in der Lage ist, sich unter bestimmten pH- oder Ionengehalt-Bedingungen des Mediums direkt durch Adsorption auf der Oberfläche zu verankern.

10. Oberfläche nach einem der Ansprüche 6 bis 9, **dadurch gekennzeichnet, dass** die Anheftungsgruppe unter den Alkyl- und halogenierten Alkylgruppen ausgewählt wird, um hydrophobe Bindungen zu realisieren.

11. Oberfläche nach Anspruch 10, **dadurch gekennzeichnet, dass** die Anheftungsgruppe ausgewählt wird unter: -CH₃, CF₃, -CH₂F, CHF₂.

12. Oberfläche nach Anspruch 6, **dadurch gekennzeichnet, dass** die Anheftung vom kovalenten Typ ist.

13. Oberfläche nach einem der Ansprüche 1 bis 12, **dadurch gekennzeichnet, dass** die organischen Verbindungen in der Lage sind, miteinander außerhalb der exponierten Gruppierung zu reagieren, um quer verlaufende Bindungen zu erzeugen.

14. Oberfläche nach einem der Ansprüche 6 bis 13, **dadurch gekennzeichnet, dass** die organische Verbindung eine Anheftungsgruppe an einem Ende und eine exponierte Gruppierung an dem anderen Ende aufweist.

15. Oberfläche nach einem der Ansprüche 6 bis 14, **dadurch gekennzeichnet, dass** die Anheftungsgruppe ausgewählt wird unter den Gruppen vom Typ Metallalkoxid, Silan, Chlorsilan, Methoxysilan und Ethoxysilan, Silanol, Silazan, vom Typ Phosphat, Hydroxy, Hydrazid, Hydrazin, Amin, Amid, Diazonium, Pyridin, Sulfat, Sulfonsäure, Carbonsäure, Boronsäure, Halogen, Säurehalogenid und Aldehyd.

16. Oberfläche nach Anspruch 15, **dadurch gekennzeichnet, dass** die Anheftungsgruppe unter den Silanen, wie Mono-, Di- oder Trichlorsilan, Mono-, Di- oder Triethoxysilanen und Mono-, Dioder Trimethoxysilanen ausgewählt wird.

17. Oberfläche nach einem der Ansprüche 1 bis 16, **dadurch gekennzeichnet, dass** die exponierte Gruppierung an die Anheftungsgruppe entweder direkt oder durch Ketten, die wenigstens ein Kohlenstoffatom und vorzugsweise 3 bis 30 Atome umfassen, gebunden ist.

18. Oberfläche nach einem der Ansprüche 1 bis 17, **dadurch gekennzeichnet, dass** der Träger wenigstens an der Oberfläche durch ein Polymer, ein Metall oder ein Metalloxid, ein Halbleiterelement, ein Oxid eines Halbleiterelements, insbesondere ein Siliciumoxid, oder eine von deren Kombinationen gebildet wird.

19. Oberfläche nach einem der Ansprüche 1 bis 18, **dadurch gekennzeichnet, dass** der Träger wenigstens an der Oberfläche aus Glas oder Siliciumoxid gebildet wird.

20. Oberfläche nach einem der Ansprüche 1 bis 19, **dadurch gekennzeichnet, dass** der Träger in Platten-, Kugel-, Faser- oder Teilchenform vorliegt.

21. Oberfläche nach Anspruch 20, **dadurch gekennzeichnet, dass** die Teilchen magnetisch, fluoreszierend oder gefärbt sind.

22. Oberfläche nach einem der Ansprüche 1 bis 20, **dadurch gekennzeichnet, dass** das Molekül mit biologischer Aktivität ausgewählt wird unter:
- den Proteinen,
- den Nukleinsäuren,
- den Lipiden,
- den Polysacchariden und deren Derivaten.

23. Oberfläche nach einem der Ansprüche 1 bis 22, **dadurch gekennzeichnet, dass** das Molekül mit biologischer Aktivität eine chemische Verbindung ist, die eine biologische Aktivität aufweist.

24. Oberfläche nach einem der Ansprüche 1 bis 23, **dadurch gekennzeichnet, dass** das Molekül mit biologischer Aktivität ausgewählt wird unter:
- den Proteinen,
- DNA,
- RNA und deren Derivaten.

25. Oberfläche nach Anspruch 24, **dadurch gekennzeichnet, dass** das Molekül mit biologischer Aktivität ausgewählt wird unter:
- den Antikörpern,
- den Antigenen,
- DNA und RNA,
- den Liganden oder deren Rezeptoren sowie deren Derivaten.

26. Verfahren zur Herstellung einer hochspezifischen Oberfläche für biologische Reaktionen nach einem der Ansprüche 1 bis 25, **dadurch gekennzeichnet, dass**:
- man auf einem festen Träger eine im wesentlichen monomolekulare und im wesentlichen kompakte Schicht von einer organischen Verbindung von langgestreckter Struktur, welche wenigstens aufweist:
. eine Anheftungsgruppe, die eine Affinität für den Träger aufweist, und
. eine exponierte Gruppierung, die keine oder nur geringe Affinität für den Träger und die Anheftungsgruppe unter den Anheftungsbedingungen aufweist, aber eine Affinität für einen Molekültyp mit biologischer Aktivität aufweist,
anheftet.

27. Mit einem Molekül mit biologischer Aktivität beschichtete Oberfläche, **dadurch gekennzeichnet, dass** sie eine hochspezifische Oberfläche für biologische Reaktionen nach einem der Ansprüche 1 bis 25 umfasst, auf welcher das Molekül mit biologischer Aktivität angeheftet ist.

28. Oberfläche nach Anspruch 27, **dadurch gekennzeichnet, dass** das Molekül mit biologischer Aktivität ausgewählt wird unter:
- den Proteinen,
- den Nukleinsäuren,
- den Lipiden,
- den Polysacchariden und deren Derivaten.

29. Oberfläche nach Anspruch 28, **dadurch gekennzeichnet, dass** die angeheftete DNA die zu einer Sequenz einer aus einer Probe zu isolierenden DNA komplementäre Sequenz umfasst.

30. Oberfläche nach Anspruch 28, **dadurch gekennzeichnet, dass** das angeheftete Protein in der Lage ist, ein aus einer Probe zu isolierendes Protein spezifisch zu erkennen und zu binden.

31. Oberfläche nach einem der Ansprüche 28 und 29, **dadurch gekennzeichnet, dass** das Molekül mit biologischer Aktivität unter Biotin, Avidin, Streptavidin oder deren Derivaten ausgewählt wird.

32. Verfahren zur Identifizierung der Anwesenheit einer DNA oder einer RNA, bei welchem man eine Oberfläche nach einem der Ansprüche 1 bis 31 einsetzt.

33. Verfahren zur Identifizierung der Anwesenheit eines Proteins oder eines Antikörpers, bei welchem man eine Oberfläche nach einem der Ansprüche 1 bis 31 einsetzt.

34. Verfahren zum Nachweis und/oder zur quantitativen Bestimmung eines Moleküls mit biologischer Aktivität in einer Probe, **dadurch gekennzeichnet, dass** man eine Oberfläche nach einem der Ansprüche 27 bis 31 einsetzt, auf welcher sich ein Molekül mit biologischer Aktivität angeheftet befindet, welches in der Lage ist, das Molekül der Probe zu erkennen, und dass der Nachweis oder die quantitative Bestimmung mit Hilfe eines fluoreszierenden oder nicht-fluoreszierenden Reagens, welches die Anwesenheit des angehefteten Moleküls detektiert, ausgeführt wird.

35. Verfahren nach Anspruch 34, **dadurch gekennzeichnet, dass** die Oberfläche schwach fluoreszierend ist und dass das Reagens fluoreszierend ist.

36. Verfahren nach Anspruch 35, **dadurch gekennzeichnet, dass** das Reagens aus Kugeln gebildet wird.

37. Verfahren nach einem der Ansprüche 34 bis 36, **dadurch gekennzeichnet, dass** der Nachweis durch Mikroskopie erfolgt.

38. Verfahren nach einem der Ansprüche 34 bis 37, **dadurch gekennzeichnet, dass** man das Produkt der Reaktion zwischen dem Molekül mit biologischer Aktivität und dem Molekül der Probe einer Beanspruchung aussetzt, um vor dem Nachweis die schlechten Paarungen zu zerstören.

39. Verfahren zum Nachweis einer DNA-Sequenz oder eines Proteins in einer Probe, **dadurch gekennzeichnet, dass**:
- man eine hochspezifische Oberfläche nach einem der Ansprüche 1 bis 31 einsetzt;
- man die Probe in Kontakt mit der Oberfläche bringt unter Bedingungen einer Bildung des DNA/DNA-, DNA/RNA- oder Protein/Protein-Hybrids,
- man, wenn das Hybrid eine Faltung aufweist, dieses durch ein(e) jegliche(s) physikalisch-chemische(s) Mittel oder Maßnahme streckt oder auseinanderzieht, um die Moleküle zu orientieren, und man die Messung oder die Beobachtung der so orientierten Moleküle vornimmt.

40. Verfahren nach einem der Ansprüche 38 und 39, **dadurch gekennzeichnet, dass** man das Hybrid oder Reaktionsprodukt durch Passage eines Meniskus in der Streckrichtung streckt oder auseinanderzieht.

41. Verfahren nach Anspruch 40, **dadurch gekennzeichnet, dass** der Meniskus ein Luft/Wassermeniskus ist.

42. Verfahren nach einem der Ansprüche 38 und 39, **dadurch gekennzeichnet, dass** das Strecken oder Auseinanderziehen durch ein elektrisches Feld, welches auf die Moleküle einwirkt, oder ein Magnetfeld, welches auf ein magnetisierbares oder magnetisches Teilchen einwirkt, bewirkt wird.

43. Verfahren nach einem der Ansprüche 38 und 39, **dadurch gekennzeichnet, dass** das Strecken oder Auseinanderziehen auf mechanischem Wege ausgeführt wird.

44. Verfahren nach einem der Ansprüche 38 bis 43, **dadurch gekennzeichnet, dass** die angeheftete DNA und die DNA der Probe auf unterschiedliche Weise "gefärbt" sind und dass man, nach dem Strecken oder Auseinanderziehen, die Lage der komplementären Sequenz bezogen auf das Ende der DNA der Probe bestimmt.

45. Verfahren nach Anspruch 44, das für die Kartierung von Genen bestimmt ist.

46. Verfahren nach einem der Ansprüche 44 bis 45, **dadurch gekennzeichnet, dass** es sich um eine ELISA- oder RIA-Methode handelt.

47. Verfahren nach einem-der Ansprüche 44 bis 45, **dadurch gekennzeichnet, dass** die Probe des Produkt einer Nukleinsäureamplifizierung ist.

48. Verfahren zur Anheftung einer Nukleinsäure oder eines Proteins auf einer Oberfläche, wie gemäß einem der Ansprüche 1 bis 25 definiert, bei welchem man eine Verankerung der DNA oder des Proteins durch Adsorption insbesondere durch ein Ende realisiert, indem man die DNA oder das Protein in einem bestimmten pH-Bereich in Kontakt mit der Oberfläche bringt.

49. Verfahren zur Anheftung von DNA auf einer Oberfläche, die Gruppierungen mit ethylenischen Doppelbindungen aufweist, nach Anspruch 48, **dadurch gekennzeichnet, dass** man die DNA bei einem pH unter 8 mit der Oberfläche in Kontakt bringt.

50. Verfahren zur Anheftung nach Anspruch 49, **dadurch gekennzeichnet, dass** die Reaktion bei einem pH zwischen 5 und 6 ausgeführt, dann bei pH 8 gestoppt wird.

51. Diagnose-Kit, **dadurch gekennzeichnet, dass** er wenigstens eine hochspezifische Oberfläche nach einem der Ansprüche 1 bis 33 umfasst.

52. Diagnose-Kit nach Anspruch 51, **dadurch gekennzeichnet, dass** er außerdem ein fluoreszierendes Reagens oder ein Reagens, das Kugeln umfasst, umfasst.

## Claims

1. Highly specific surface for biological reactions, **characterized in that** it contains a support having at the surface at least one essentially compact layer of an organic compound having, outside the layer, an exposed group containing an ethylenic double bond having affinity for one type of molecule with biological activity under certain pH or ionic content conditions, the other elements of the layer being essentially inaccessible for the said molecules under the said reaction conditions.

2. Surface according to Claim 1, **characterized in that** the support is a solid support.

3. Highly specific surface according to either of Claims 1 and 2, **characterized in that** the exposed group is a vinyl group.

4. Surface according to one of Claims 1 to 3, **characterized in that** it is obtained by polymerization of at least one monomer generating at the surface of the polymer with an ethylenic double bond the said exposed group, by deposition of polymer on the support or by partial depolymerization of the surface of a polymer in order to generate the said exposed group.

5. Surface according to Claim 4, **characterized in that** the polymer is a polyolefin or a polyene derivative.

6. Highly specific surface according to one of Claims 1 to 5, **characterized in that** it contains
- on a support, a substantially monomolecular and compact layer of an organic compound of elongated structure having at least:
• an attachment group having affinity for the support, and
• an exposed group containing an ethylenic double bond, having little or no affinity for the said support and the said attachment group under the attachment conditions, but having affinity for one type of biological molecule.

7. Surface according to Claim 6, **characterized in that** the attachment is of the noncovalent type.

8. Surface according to Claim 7, **characterized in that** the attachment is carried out by hydrophilic or hydrophobic interactions.

9. Surface according to Claim 8, **characterized in that** the biological molecule is capable of anchoring directly by adsorption on the said surface under certain conditions of pH or of ionic content of the medium.

10. Surface according to one of Claims 6 to 9, **characterized in that** the attachment group is chosen from alkyl and halogenated alkyl groups in order to have hydrophobic bonds.

11. Surface according to Claim 10, **characterized in that** the attachment group is chosen from: -CH₃, CF₃, -CH₂F, CHF₂.

12. Surface according to Claim 6, **characterized in that** the attachment is of the covalent type.

13. Surface according to one of Claims 1 to 12, **characterized in that** the organic compounds are capable of reacting with each other excluding the exposed group in order to create cross-linkages.

14. Surface according to one of Claims 6 to 13, **characterized in that** the organic compound has an attachment group at one end and an exposed group at the other end.

15. Surface according to one of Claims 6 to 14, **characterized in that** the attachment group is chosen from the groups of the metal alkoxide type, such as silane, chlorosilane, methoxysilane and ethoxysilane, silanol, silazane, of the phosphate, hydroxyl, hydrazide, hydrazine, amine, amide, diazonium, pyridine, sulphate, sulphonic, carboxylic, boronic, halogen, acid halide and aldehyde type.

16. Surface according to Claim 15, **characterized in that** the attachment group is chosen from the silanes such as mono-, di- or trichlorosilane, mono-, di- or triethoxysilanes and mono, di- or trimethoxysilanes.

17. Surface according to one of Claims 1 to 16, **characterized in that** the exposed group is either linked directly to the attachment group, or by chains containing at least one carbon atom, and preferably 3 to 30 atoms.

18. Surface according to one of Claims 1 to 17, **characterized in that** the support consists, at least at the surface, of a polymer, a metal or a metal oxide, a semiconductor element, an oxide of a semiconductor element, especially a silicon oxide, or a combination thereof.

19. Surface according to one of Claims 1 to 18, **characterized in that** the support consists, at least at the surface, of glass or silica.

20. Surface according to one of Claims 1 to 19, **characterized in that** the support is in the form of a plate, beads, fibre or particles.

21. Surface according to Claim 20, **characterized in that** the said particles are magnetic, fluorescent or coloured.

22. Surface according to one of Claims 1 to 20, **characterized in that** the molecule with biological activity is chosen from:
- proteins,
- nucleic acids
- lipids,
- polysaccharides and derivatives thereof.

23. Surface according to one of Claims 1 to 22, **characterized in that** the molecule with biological activity is a chemical compound having a biological activity.

24. Surface according to one of Claims 1 to 23, **characterized in that** the molecule with biological activity is chosen from:
- proteins,
- DNA,
- RNA and derivatives thereof.

25. Surface according to Claim 24, **characterized in that** the molecule with biological activity is chosen from:
- antibodies
- antigens
- DNA and RNA
- ligands or their receptors as well as derivatives thereof.

26. Process for preparing a highly specific surface for biological reactions according to one of Claims 1 to 25, **characterized in that**:
- a substantially monomolecular and essentially compact layer of an organic compound of elongated structure having at least:
• an attachment group having an affinity for the support, and
• an exposed group having no or little affinity for the said support and the attachment group under the attachment conditions, but having an affinity for one type of molecule having biological activity,
is attached onto a solid support.

27. Surface coated with a molecule with biological activity, **characterized in that** it contains a highly specific surface for biological reactions according to one of Claims 1 to 25 on which the said molecule with biological activity is attached.

28. Surface according to Claim 27, **characterized in that** the molecule with biological activity is chosen from:
- proteins,
- nucleic acids
- lipids,
- polysaccharides and derivatives thereof.

29. Surface according to Claim 28, **characterized in that** the attached DNA contains the sequence complementary to a DNA sequence to be isolated from a sample.

30. Surface according to Claim 28, **characterized in that** the attached protein is capable of specifically recognizing and attaching a protein to be isolated from a sample.

31. Surface according to either of Claims 28 and 29, **characterized in that** the said molecule with biological activity is chosen from biotin, avidin, streptavidin or derivatives thereof.

32. Process for identifying the presence of a DNA or an RNA using a surface according to one of Claims 1 to 31.

33. Process for identifying the presence of a protein or an antibody using a surface according to one of Claims 1 to 31.

34. Process for detecting and/or assaying a molecule with biological activity in a sample, **characterized in that** a surface according to one of Claims 27 to 31 is used on which is attached a molecule with biological activity capable of recognizing the sample molecule, and **in that** the detection or assay are carried out using a reagent, fluorescent or otherwise, which detects the presence of the attached molecule.

35. Process according to Claim 34, **characterized in that** the surface has a low fluorescence and **in that** the reagent is fluorescent.

36. Process according to Claim 35, **characterized in that** the reagent consists of beads.

37. Process according to one of Claims 34 to 36, **characterized in that** the detection is performed by microscopy.

38. Process according to one of Claims 34 to 37, **characterized in that** the product of the reaction between the molecule with biological activity and the sample molecule is subjected to a stress so as to destroy the mismatches before the detection.

39. Process for detecting a DNA sequence or a protein in a sample, **characterized in that**:
- a highly specific surface according to one of Claims 1 to 31 is used:
- the sample is brought into contact with the surface under conditions for forming the DNA/DNA, DNA/RNA or protein/protein hybrid,
- the hybrid having been labelled, it is stretched by any physicochemical means in order to orientate the molecules and to carry out the measurement or observation of the molecules thus orientated.

40. Process according to either of Claims 38 and 39, **characterized in that** the hybrid or reaction product is stretched by passage of a meniscus in the direction of the stretching.

41. Process according to Claim 40, **characterized in that** the meniscus is an air/water meniscus.

42. Process according to either of Claims 38 and 39, **characterized in that** the stretching is carried out by an electric field acting on the molecules or a magnetic field acting on a magnetizable or magnetic particle.

43. Process according to either of Claims 38 and 39, **characterized in that** the stretching is carried out mechanically.

44. Process according to one of Claims 38 to 43, **characterized in that** the attached DNA and the sample DNA are "coloured" differently, and **in that**, after stretching, the position of the complementary sequence relative to the end of the sample DNA is measured.

45. Process according to Claim 44, intended for the mapping of genes.

46. Process according to one of Claims 44 to 45, **characterized in that** it is an ELISA or RIA method.

47. Process according to one of Claims 44 to 45, **characterized in that** the sample is the product of a nucleic acid amplification.

48. Process for attaching a nucleic acid or a protein on a surface as defined according to one of Claims 1 to 25, in which anchoring is carried out by adsorption of the DNA or of the protein, especially by one end by bringing the DNA or the protein into contact with the surface in a determined pH region.

49. Process for attaching DNA on a surface having groups with ethylenic double bond according to Claim 48, **characterized in that** the DNA is brought into contact with the surface at a pH of less than 8.

50. Process of attachment according to Claim 49, **characterized in that** the reaction is carried out at a pH of between 5 and 6, and is then stopped at pH 8.

51. Diagnostic kit, **characterized in that** it comprises at least one highly specific surface according to one of Claims 1 to 33.

52. Diagnostic kit according to Claim 51, **characterized in that** it contains, in addition, a fluorescent reagent or a reagent containing beads.
